# DEMANDE DE BREVET EUROPEEN

(11) **EP 1 870 047 A1**
(43) Date de publication de la demande: **26.12.2007**
(21) Numéro de dépôt: 07110829.4
(22) Date de dépôt: 22.06.2007
(51) Int. Cl.: A61B 17/68

(54) **Broche de fixation osseuse**

(30) Priorité: 22.06.2006 FR 0605604
(71) Demandeur: Textile Hi-Tec (T.H.T.), 34220 Verreries de Moussans (FR)
(72) Inventeur: Houard, William, 81270, LABASTIDE-ROUAIROUX (FR)
(74) Mandataire: Hennion, Jean-Claude

(57) **Abrégé**

La broche de fixation osseuse (1) comporte un corps cylindrique (2) terminé par une extrémité distale (3) de configuration conique et présente sur sa surface extérieure des éléments anti-migratoires, constitués par une pluralité de crans (4,4'), répartis sur la longueur du corps cylindrique (2). Chaque cran (4) forme une surépaisseur qui s'étend en arc de cercle sur une partie transversale de la périphérie du corps cylindrique (2) et a une configuration inclinée vers l'extrémité distale (3).

De préférence deux crans, de préférence deux ou trois crans, adjacents (4,4') ont une disposition alternée, en sorte que leurs arcs de cercle cumulés occupent toute la périphérie du corps cylindrique.

## Description

La présente invention concerne le domaine chirurgical lié à la réparation osseuse, notamment en cas de fracture. Elle concerne plus particulièrement une broche de fixation osseuse destinée à être insérée dans l'os par le praticien de part et d'autre d'une fracture.

Les outils mis à la disposition du chirurgien pour l'ostéosynthèse sont multiples : vis, broches, ancres et plaques.

S'agissant plus précisément des broches de fixation osseuse, il en existe un très grand nombre de modèles. Elles ont généralement un corps cylindrique de révolution, terminé par une extrémité distale en pointe permettant sa pénétration dans l'os.

Contrairement aux vis, les broches ne comportent pas sur leur surface extérieure de filetage en hélice, mais elles peuvent néanmoins comporter des éléments anti-migratoire, visant à empêcher le déplacement non souhaité de la broche une fois que celle-ci est implantée dans l'os. Dans le document W0.97/37603, les éléments anti-migratoires se présentent soit sous la forme de pointes soit la forme d'une succession de filets circulaires répartis sur toute la longueur de la broche.

Ce mode de réalisation d'une broche avec une succession de filets circulaires également répartis sur toute la longueur de la broche n'est aucunement explicité dans le texte de ce document W0.97/37603, exception faite de la représentation qui en est faite sur la figure 22.

Ceci étant, il apparaît que ce mode de réalisation peut présenter un inconvénient majeur. La présence des filets circulaires constitue une augmentation localisée importante du diamètre de la broche, ce qui peut poser une difficulté pour la pénétration de la broche dans l'alésage, préalablement percé par le praticien dans la partie osseuse à l'aide d'un foret dont le diamètre est sensiblement celui du corps cylindrique de la broche.

Le but de la présente invention est de pallier les inconvénients précités des broches antérieures en proposant une broche de fixation osseuse qui comporte, de manière connue, un corps cylindrique terminé par une extrémité distale de configuration conique et présentant sur sa surface extérieure des éléments anti-migratoires. De manière caractéristique, selon la présente invention, les éléments anti-migratoires sont constitués par une pluralité de crans, répartis sur la longueur du corps cylindrique, chaque cran formant une surépaisseur qui d'une part s'étend en arc de cercle sur une partie transversale de la périphérie du corps cylindrique et qui d'autre part a une configuration inclinée vers l'extrémité distale.

Grâce à ces caractéristiques spécifiques, l'implantation de la broche dans la partie osseuse se fait dans des conditions beaucoup plus douces.

Selon une variante de réalisation, l'arc de cercle formé par chaque cran est de l'ordre de 180°. En d'autres termes, chaque cran occupe sensiblement la moitié de la périphérie du corps cylindrique.

Dans un mode de réalisation de cette même variante, au moins deux crans adjacents ont une disposition alternée, en sorte que leurs arcs de cercle cumulés occupent toute la périphérie du corps cylindrique. Ainsi, selon cette configuration particulière, la broche a bien la même section transversale au niveau de chaque cran, mais la disposition alternée des crans sur la périphérie du corps cylindrique permet d'obtenir un équilibre de l'effet anti-migratoire.

Selon une variante de réalisation, l'angle d'inclinaison de la face inclinée de chaque cran est de l'ordre de 30°. Cet angle permet une pénétration en douceur de la broche dans l'alésage précédemment percé par le praticien à l'aide d'un foret dont le diamètre correspond sensiblement à celui du seul corps cylindrique.

De préférence, la broche étant destinée aux ostéosynthèses de petits fragments osseux, le corps cylindrique a une section circulaire dont le diamètre fait de l'ordre de 2 à 3 mm et chaque cran a une hauteur maximale de l'ordre de 0,2 à 0,4 mm.

Dans un mode précis de réalisation, donné à titre non exhaustif, la broche a une longueur totale de l'ordre de 80 mm et comporte treize crans espacés l'un de l'autre de l'ordre de 5 mm.

Dans une variante de réalisation, le corps cylindrique a une portion d'extrémité proximale, limitée en longueur, de diamètre D2 plus réduit, ladite portion d'extrémité étant destinée à servir au calage de la broche lors de sa coopération avec un poussoir coulissant.

De préférence, la broche de fixation osseuse selon la présente invention est formée d'un seul bloc dans un matériau biodégradable et/ou résorbable, choisi dans le groupe suivant :
- polylactide, polyglycolide, poly-ε-caprolactone,
- polyhydroxy butyrate, polyhydroxy valérate,
- poly carbonates et assimilés,
- cellulose, polysaccharides, amidon,
leurs homopolymères, leurs copolymères et leurs dérivés.

De préférence dans ce cas, le matériau biodégradable et/ou résorbable comporte une charge, en proportion en poids de 5 à 60 %, qui est une poudre ou une nano poudre d'un constituant choisi parmi le groupe : hydroxy apatite et/ou de phosphate de calcium, notamment de β tricalcium phosphate, et/ou d'hydrogéno phosphate de calcium et/ou de carbonate de calcium et/ou d'hydrogéno carbonate de calcium.

La présente invention a également pour objet un ensemble chirurgical de pose de broches de fixation osseuse comprenant :
- au moins une broche ayant la structure et la composition définies ci-dessus,
- au moins un foret de percement de l'os adapté au diamètre d'une broche,
- un tube d'introduction, présentant un canal central dont le diamètre permet de loger la broche et
- un poussoir coulissant, apte à se déplacer dans ledit canal central en prenant appui sur l'extrémité proximale de la broche ou la portion d'extrémité du corps cylindrique de la broche et en repoussant celle-ci en sorte que la broche soit évacuée du tube d'introduction.

Ainsi, le praticien a à sa disposition tous les outils qui lui permettent d'effectuer la pose de la broche de fixation osseuse de la présente invention.

Lorsque l'ensemble chirurgical comporte un jeu de plusieurs broches de diamètres différents, il comporte bien entendu les forets de percement correspondant aux diamètres des broches en question. De préférence, dans ce cas, toutes les broches ont en partie proximale le même diamètre, qu'il s'agisse de l'extrémité proximale elle-même ou de la portion d'extrémité proximale de section réduite, l'extrémité distale du poussoir coulissant comporte un logement ajusté pour ladite partie proximale, de telle sorte que le même tube d'introduction et le même poussoir coulissant puisse servir à l'implantation de toutes les broches.

La présente invention sera mieux comprise à la lecture de la description qui va être faite d'un exemple préféré de réalisation d'un ensemble chirurgical de pose d'une broche de fixation osseuse à crans transversaux en arc de cercle, illustré par le dessin annexé dans lequel :
- la figure 1 est une représentation schématique en perspective de la broche,
- la figure 2 est une représentation schématique en perspective d'une partie de l'ensemble chirurgical avec la broche de fixation osseuse placée dans le tube d'introduction avec le poussoir coulissant en position d'actionnement et
- la figure 3 est une représentation schématique en coupe longitudinale de la broche, de l'extrémité avant du poussoir coulissant et l'extrémité arrière du tube d'introduction.

L'objet de la présente invention fait partie des outils qui sont à la disposition du chirurgien pour pratiquer une ostéosynthèse, c'est-à-dire une intervention ayant pour but de réunir mécaniquement les fragments osseux d'une fracture. Cet outil est une broche 1 dont le corps cylindrique 2 est terminé par une extrémité distale 3 de configuration conique à bout 3a arrondi.

Cette broche 1 est munie d'éléments anti-migratoires qui sont disposés sur la surface extérieure du corps cylindrique 2. En l'occurrence, selon la présente invention, ces éléments anti-migratoires sont des crans 4 qui sont répartis sur toute la longueur du corps cylindrique 2. Chaque cran 4 est disposé transversalement par rapport à la direction longitudinale AA' de la broche - c'est-à-dire perpendiculairement à cette direction AA' - , se présentant sous la forme d'une surépaisseur qui s'étend sur la surface extérieure du corps cylindrique 2 selon un arc de cercle inférieur à 360°, c'est-à-dire ne faisant pas toute la périphérie du corps cylindrique. De plus chaque cran 4 est distant du cran adjacent d'une distance donnée, qui de préférence est une distance d constante pour une broche donnée.

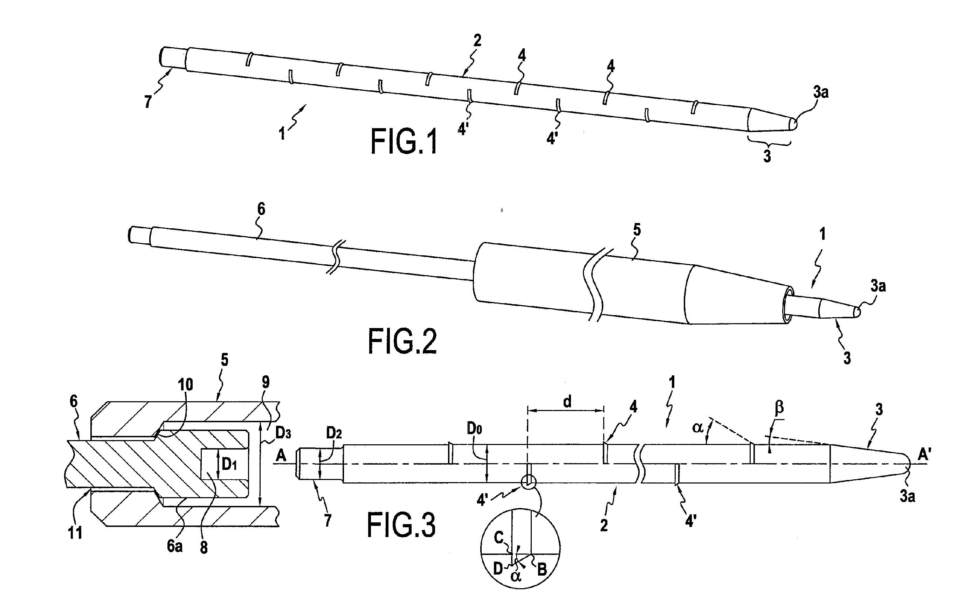

Dans l'exemple préféré de réalisation qui est illustré à la figure 1, chaque cran 4 occupe un arc de cercle de 180°, c'est-à-dire la moitié de la périphérie du corps cylindrique 2. De plus deux crans adjacents sont décalés angulairement également de 180°, ce qui fait que deux crans successifs 4, 4' occupent cumulativement toute la périphérie du corps cylindrique. Par ailleurs, comme cela apparaît plus clairement à l'examen de la figure 3, la surépaisseur formée par chaque cran a une section longitudinale en forme de triangle rectangle BCD dont l'hypoténuse BD est inclinée vers l'extrémité distale 3 d'un angle α par rapport à la face extérieure du corps cylindrique, correspondant au côté BC. Dans l'exemple illustré, cet angle α est de 30°, tandis que l'angle β d'inclinaison de l'extrémité distale conique est de 15°.

Dans ce même exemple précis de réalisation, la broche 1 a une longueur totale de l'ordre de 80 mm et comporte treize crans 4 espacés les uns des autres d'une distance d de l'ordre de 5 mm. Le corps cylindrique 2 a une section circulaire dont le diamètre est de 2,5 mm et l'épaisseur maximale de chaque cran - correspondant au côté CD - fait de l'ordre de 0,2 mm.

Avant d'implanter la broche 1 dans la partie osseuse, le chirurgien perce avec un foret non représenté un alésage dont le diamètre correspond sensiblement au diamètre D0 du corps cylindrique 2. Certes pour la pénétration de la broche 1 dans l'alésage il est nécessaire de forcer le passage au niveau des crans 4,4'. Cependant la configuration inclinée desdits crans vers l'extrémité distale de la broche et le fait que chaque cran n'occupe qu'un arc de cercle facilite grandement cette pénétration sans détérioration de la partie osseuse constituant la paroi de l'alésage.

Bien sûr la présente invention n'est pas limitée au mode précis de réalisation qui vient d'être décrit. Les crans pourraient occuper un arc de cercle différent de 180°, par exemple 120° avec une disposition alternée non plus sur deux mais sur trois crans adjacents de sorte que les arcs de cercle cumulés des trois crans adjacents occupent toute la périphérie du corps cylindrique. Il importe en effet qu'après l'implantation de la broche dans la partie osseuse, celle-ci n'ait pas tendance à se déplacer. La disposition alternée sur plusieurs crans de telle sorte que les arcs de cercle cumulés desdits crans occupent toute la périphérie du corps cylindrique permet d'obtenir un effet anti-migratoire homogène permettant d'atteindre le but recherché c'est-à-dire la stabilité de la broche dans l'os.

La broche de fixation osseuse peut être une broche métallique qui reste à demeure pendant toute la réparation osseuse et que l'on va extraire à l'issue de celle-ci. De préférence, pour éviter ce geste complémentaire, la broche de fixation osseuse 1 est formée d'un seul bloc dans un matériau biodégradable et/ou résorbable, choisi dans le groupe suivant :
- polylactide, polyglycolide, poly-ε-caprolactone,
- polyhydroxy butyrate, polyhydroxy valérate,
- poly carbonates et assimilés,
- cellulose, polysaccharides, amidon,
leurs homopolymères, leurs copolymères et leurs dérivés.

Dans le mode précis de réalisation décrit ci-dessus, ladite broche a été réalisée en acide poly lactique.

Pour améliorer la prolifération osseuse lors de la résorption de la broche, il est également souhaitable que le matériau biodégradable et/ou résorbable soit chargé, dans une proportion de 5 à 60 %, de poudre ou de nano poudre d'un constituant choisi parmi le groupe suivant : hydroxy apatite et/ou de phosphate de calcium, notamment de β tricalcium phosphate, et/ou d'hydrogéno phosphate de calcium et/ou de carbonate de calcium et/ou d'hydrogéno carbonate de calcium.

Pour assister le praticien, il est proposé un ensemble chirurgical de pose de broche qui comprend, outre une broche ou un jeu plusieurs broches telles que décrites ci-dessus, un foret ou un jeu de plusieurs forets de percement, chacun ayant un diamètre correspondant sensiblement au diamètre du corps cylindrique d'une broche, un tube d'introduction 5, présentant un canal central 9 dont le diamètre D3 permet de loger la broche 1 ou chacune des broches du jeu et un poussoir coulissant 6 apte à prendre appui sur la partie proximale de la broche 1 et déplacer celle-ci en sorte que la broche soit évacuée du tube d'introduction 5.

Pour améliorer le positionnement du poussoir coulissant 6 par rapport à la partie proximale de la broche, le poussoir coulissant 6 présente à son extrémité distale une portion évidée cylindrique 8 dont le diamètre intérieur D1 est égal ou très légèrement supérieur au diamètre extérieur D2 de ladite partie proximale, qui peut être soit l'extrémité proximale du corps cylindrique de la broche, soit, comme dans l'exemple de la figure 3, une portion d'extrémité 7 ayant un diamètre D2 inférieur à celui D0 du corps cylindrique 2.Ainsi ladite partie proximale 7 de la broche vient s'emmancher dans la portion évidée 8 de l'extrémité distale du poussoir coulissant 6. De plus ladite extrémité distale 6a du poussoir coulissant 6 comporte un épaulement annulaire 10 d'une part permettant le coulissement ajusté du poussoir coulissant 6 dans le canal 9 et d'autre part faisant office de butée empêchant la sortie du poussoir coulissant 6 de l'ouverture proximale 11 du tube d'introduction 5. Il est ainsi possible d'obtenir un déplacement rectiligne continu de la broche 1 lors de son implantation dans l'alésage percé dans l'os, quel que soit le diamètre de celle-ci, en utilisant le même tube d'introduction 5 et le même poussoir coulissant 6.

## Revendications

1. Broche de fixation osseuse (1) comportant un corps cylindrique (2) terminé par une extrémité distale (3) de configuration conique et présentant sur sa surface extérieure des éléments anti-migratoires, **caractérisée en ce que** les éléments anti-migratoires sont constitués par une pluralité de crans (4,4'), répartis sur la longueur du corps cylindrique (2), chaque cran (4) formant une surépaisseur qui d'une part s'étend en arc de cercle sur une partie de la périphérie du corps cylindrique (2) qui est transversale par rapport à la direction longitudinale (AA') de la broche et qui d'autre part a une configuration inclinée vers l'extrémité distale (3).

2. Broche selon la revendication 1 **caractérisée en ce que** l'arc de cercle formé par chaque cran (4,4') est de l'ordre de 180°.

3. Broche selon la revendication 2 **caractérisée en ce qu'**au moins deux crans, de préférence deux ou trois crans, adjacents (4,4') ont une disposition alternée, en sorte que leurs arcs de cercle cumulés occupent toute la périphérie du corps cylindrique.

4. Broche selon l'une des revendications 1 à 3 **caractérisée en ce que** l'angle d'inclinaison (α) de la face inclinée de chaque cran (4) est de l'ordre de 30°.

5. Broche selon l'une des revendications 1 à 4 **caractérisée en ce que** le corps cylindrique (2) a une section circulaire dont le diamètre (D0) fait de l'ordre de 2 à 3 mm et chaque cran a une hauteur maximale de l'ordre de 0,2 à 0,4 mm.

6. Broche selon l'une des revendications 1 à 5 **caractérisée en ce qu'**elle a une longueur totale de l'ordre de 80 mm et comporte treize crans (4,4') espacés l'un de l'autre de l'ordre de 5 mm.

7. Broche selon l'une des revendications 1 à 6 **caractérisée en ce que** le corps cylindrique (2) a une portion d'extrémité proximale (7), limitée en longueur, de diamètre (D2) plus réduit, ladite portion d'extrémité étant destinée à servir au calage de la broche (1) lors de sa coopération avec un poussoir coulissant (6).

8. Broche selon l'une des revendications 1 à 7 **caractérisée en ce qu'**elle est formée d'un seul bloc dans un matériau biodégradable et/ou résorbable, choisi dans le groupe suivant :
- polylactide, polyglycolide, poly-ε-caprolactone,
- polyhydroxy butyrate, polyhydroxy valérate,
- poly carbonates et assimilés,
- cellulose, polysaccharides, amidon,
leurs homopolymères, leurs copolymères et leurs dérivés.

9. Broche selon la revendication 8 **caractérisée en ce que** le matériau biodégradable et/ou résorbable comporte une charge, en proportion en poids de 5 à 60 %, qui est une poudre ou une nano poudre d'un constituant choisi parmi le groupe : hydroxy apatite et/ou de phosphate de calcium, notamment de β tricalcium phosphate, et/ou d'hydrogéno phosphate de calcium et/ou de carbonate de calcium et/ou d'hydrogéno carbonate de calcium.

10. Ensemble chirurgical de pose de broches de fixation osseuse comprenant :
- au moins une broche (1) selon l'une des revendications 1 à 9,
- au moins un foret de percement de l'os adapté au diamètre d'une broche,
- un tube d'introduction (5), présentant un canal central (9) dont le diamètre (D3) permet de loger la broche (1) et
- un poussoir coulissant (6), apte à se déplacer dans ledit canal central (9) en prenant appui sur l'extrémité proximale de la broche (1) ou la portion d'extrémité (7) du corps cylindrique (2) de la broche (1) et en repoussant celle-ci en sorte que la broche (1) soit évacuée du tube d'introduction.

11. Ensemble chirurgical selon la revendication 10 **caractérisé en ce qu'**il comporte un jeu de plusieurs broches de diamètres différents et des forets de percement correspondant aux diamètres desdites broches et **en ce que** toutes les broches ont en partie proximale le même diamètre (D2), qu'il s'agisse de l'extrémité proximale elle-même ou de la portion d'extrémité proximale (7) de section réduite et **en ce que** l'extrémité distale (6a) du poussoir coulissant (6) comporte un logement (8) ajusté pour ladite partie proximale, de telle sorte que le même tube d'introduction (5) et le même poussoir coulissant (6) puissent servir à l'implantation de toutes les broches (1).
